(19) Europäisches Patentamt
European Patent Office
Office européen des brevets

(11) Publication number: **0 242 986 A1**

# (12) EUROPEAN PATENT APPLICATION

(21) Application number: **87302388.1**

(22) Date of filing: **19.03.87**

(51) Int. Cl.⁴: **C07C 119/042**, C07C 103/30, C07C 127/15, C07D 207/26, C07D 211/18, C07D 265/30

The title of the invention has been amended (Guidelines for Examination in the EPO, A-III, 7.3).

(30) Priority: **20.03.86 GB 8606936**

(43) Date of publication of application: **28.10.87 Bulletin 87/44**

(84) Designated Contracting States: **BE CH DE FR GB IT LI NL**

(71) Applicant: **IMPERIAL CHEMICAL INDUSTRIES PLC**
**Imperial Chemical House Millbank**
**London SW1P 3JF(GB)**

(72) Inventor: **Chambers, Richard Dickinson**
**5 Aykley Green**
**Whitesmocks Durham DH1 4LN(GB)**
Inventor: **Jones, Stephen Leslie**
**Hazeldene Bottom Road**
**Radnage High Wycombe Bucks HP14 4EQ(GB)**
Inventor: **Grievson, Brian**
**7 Silverton Road**
**Guisborough Cleveland(GB)**

(74) Representative: **Stephenson, Kenneth et al**
**Imperial Chemical Industries PLC Legal Department: Patents PO Box 6 Bessemer Road**
**Welwyn Garden City Herts, AL7 1HD(GB)**

## (54) Fluorinated organic nitrogen compounds.

(57) An organic fluorine compound of the formula:

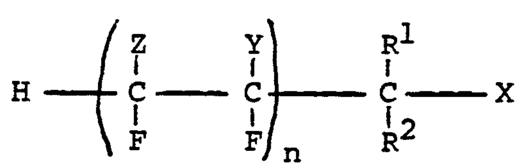

wherein X represents an isocyanato, optionally substituted amino or optionally substituted amido group;
$R^1$ represents hydrogen or an optionally substituted alkyl radical;
$R^2$ represents hydrogen or an alkyl radical;
Y represents F and Z represents F, Cl or $CF_3$ or Y and Z together form a $-(CF_2)_m-$ chain wherein m represents an integer from 2 to 4, and
n represents a positive integer which must be 1 when Z is C or $CF_3$.

EP 0 242 986 A1

## ORGANIC COMPOUNDS

This invention relates to organic compounds and more particularly to organic fluorine compounds and to a method for their preparation.

According to the invention, there are provided organic fluorine compounds of the formula:

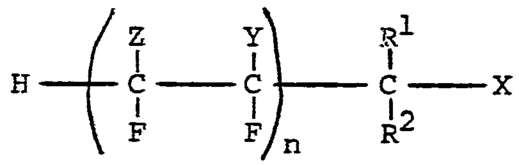

(1)

wherein X represents an isocyanato, optionally substituted amino or optionally substituted amido group;
$R^1$ represents hydrogen or an optionally substituted alkyl radical;
$R^2$ represents hydrogen or an alkyl radical;
Y represents F and Z represents F, Cl or $CF_3$ or Y and Z together form a $-(CF_2)_m-$ chain wherein m represents an integer from 2 to 4, and
n represents a positive integer which must be 1 when Z is Cl or $CF_3$.

When X in Formula 1 is an isocyanato group, optionally substituted alkyl radicals which may be represented by $R^1$ include isocyanato-alkyl with the possibility of a further substituent of the formula:

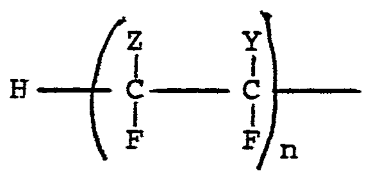

(2)

on a carbon atom adjacent to an isocyanato group.

Compounds of Formula 1 in which X is an optionally substituted amino group include compounds of the formula:

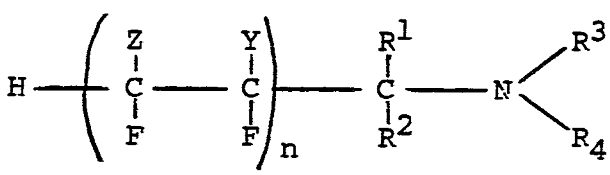

(3)

wherein $R^1$, $R^2$, Y, Z and n have the same meanings as in Formula 1, $R^3$ and $R^4$ represent optionally substituted alkyl radicals or $R^3$ and $R^4$ together, or $R^3$ and $R^1$ together, represent an alkylene chain which may be interrupted by a hetero-atom.

In the compounds of Formula 3, the substituents present on $R^1$, $R^3$ or $R^4$ may be such that the compound contains more than one amino group and/or more than one substituent of Formula 2. When $R^3$ and $R^4$ together, or $R^3$ and $R^1$ together, form an optionally interrupted alkylene chain they may, for example, together with the nitrogen atom, form a pyrrolidine, piperidine or morpholine ring.

Compounds of Formula 1 in which X is an optionally substituted amido group include compounds of the formula:

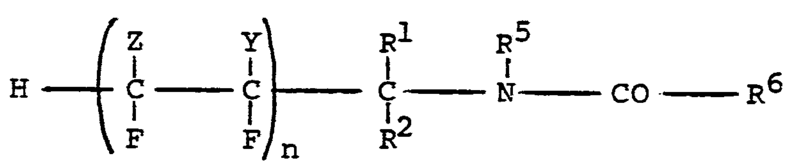

(4)

wherein $R^1$, $R^2$, Y, Z and n have the same meanings as in Formula 1, $R^5$ represents a hydrogen or an

optionally substituted alkyl radical and $R^6$ represents an optionally substituted alkyl, aryl or dialkylamino radical or $R^5$ and $R^6$ together, or $R^5$ and $R^1$ together, represent an alkylene chain which may be interrupted by a hetero-atom.

In the compounds of Formula 4, the substituents present on $R^1$, $R^5$ and $R^6$ may be such that the compound contains more than one amido group and/or more than one substituent of Formula 2. When $R^1$ and $R^5$ together form an optionally interrupted alkylene chain they may, for example, together with the nitrogen atom, form a pyrrolidine, piperidine or morpholine ring whilst $R^5$ and $R^6$ together with the nitrogen atom and carbonyl group may form a pyrrolidinone ring.

Alkyl radicals which may be present in the compounds of Formula 1, including specifically the compounds of Formulae 3 and 4, include particularly $C_{1-4}$ alkyl radicals but also higher alkyl radicals containing up to 12 or even more carbon atoms. Aryl radicals which may be represented by $R^6$ include naphthyl and especially phenyl radicals which may carry one or more substituents.

The invention also provides a method for the preparation of compounds of Formula 1 which comprises the free radical addition of a compound of the formula:

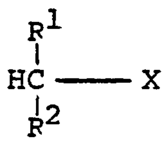

(5)

and a fluoro-olefin of the formula:

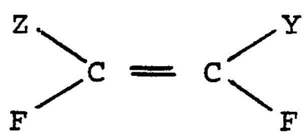

(6)

wherein X, $R^1$, $R^2$, Y and Z have the same meanings as in Formula 1.

Compounds of Formula 5 in which X is an isocyanato group include mono-isocyanates, diisocyanates and higher polyisocyanates. Examples of such compounds include methyl isocyanate, ethyl isocyanate and hexamethylene diisocyanate. Compounds of Formula 5 in which X is an amino group include mono-amines, diamines and higher polyamines. Examples of such compounds include N-methylpiperidine, N-methylmorpholine, N,N'-dimethylpiperazine and N,N,N',N'-tetramethyldiaminomethane. Compounds of Formula 5 in which X is an amido group include mono-and polyamides and ureas, for example dimethylformamide, dimethylacetamide, dimethylbenzamide, N-acetylpiperidine, N-acetylmorpholine, N-methylpyrrolidinone, polyvinylpyrrolidinone, poly(hexamethyleneadipamide) and N,N,N',N'-tetramethylurea.

Compounds of Formula 6 include tetrafluoroethene, hexafluoropropene, chlorotrifluoroethene, hexafluorocyclobutene, octafluorocyclopentene and decafluorocyclohexene.

The reaction between the compound of Formula 5 and the compound of Formula 6 is a free radical addition and accordingly is performed under conditions in which free radicals are generated. Such conditions have been fully described in the prior art and include the use of known free radical generators, for example peroxides such as di-t-butyl peroxide and dibenzoyl peroxide as well as ultra-violet and gamma radiation.

The reaction may be carried out at normal or elevated temperatures. Solvents are usually not necessary but may be used if desired. The reaction product may be a mixture of the various possible addition products that can be formed, the composition of the mixture being influenced by the molar ratio of reactants employed. Separation of the products from each other and from any remaining starting materials may be effected using conventional techniques.

The compounds of Formula 1 are valuable chemical intermediates which may be used for introducing fluorocarbon residues into polymers, resins and other end products where the present of fluorine may be desirable. Thus, the fluorinated diisocyanates of Formula 1 may be converted, using known methods, to fluorinated polyurethanes, polyureas, polyisocyanurates and polycarbodiimides.

The invention is illustrated but not limited by the following Examples:

Example 1

Ethylisocyanate (5.0g, 71.7mmol) was placed in a Carius tube (ca 100cm$^3$) and degassed. To this was added under vacuum hexafluoropropene (3.35g, 22.3mmol) and the tube sealed and irradiated with x-rays to a dose of 1.6 x $10^7$rad. On opening, the product mixture was shown to contain 2,2,3,4,4,4-hexafluoro-1-methylbutylisocyanate (14%) by Gas Chromatography.

Example 2

Ethylisocyanate (32.83g, 0.462mmol) and ditertiarybutyl peroxide (3.00g) were placed in a steel autoclave (ca 160cm$^3$) and to this added under vacuum hexafluoropropene (30.57g, 0.206mol) and then heated to 140°C for 24 hours. The product mixture was distilled to give 2,2,3,4,4,4-hexafluoro-1-methylbutylisocyanate (7.18g, 16%): boiling point, 128-129°C: Analysis, Found: C, 33.56%; H, 2.37; N, 7.04; F, 46.03; $C_6H_5F_6NO$ requires: C, 32.6%; H, 2.3; N, 6.3; F, 51.6. Infra Red, 2260 (vs) (-NCO) 1170 (vs) (C-F). Mass Spectrum (Chemical ionisation) m/e, 222 (44%) (M+ 1).

Example 3

A mixture of N-methylpiperidine (6.33g, 64mmol) and hexafluoropropene (2.99g, 20mmol) was irradiated with gamma rays. The product was transferred under vacuum to give a volatile component (6.91g) and leave a liquid (1.26g). Preparative GLC (column K, 120°C) of the liquid gave N-(2,2,3,4,4,4-hexafluorobutyl)-piperidine, (1.7% by GLC, column K, 140°C); (insufficient for analysis); N-methyl-2-(1,1,2,3,3,3-hexafluoropropyl)piperidine, (2.3% by GLC, column K, 140°C); (insufficient for analysis); and N-(2,2,3,4,4,4-hexafluorobutyl)-2-(1,1,2,3,3,3-hexafluoropropyl)piperidine, (18% by GLC, column K, 140°C); b.p. 220°C (Siwoloboff); (Found: C, 36.6%; H, 2.9%; N, 3.2%; P, 53.2%. $C_{12}H_{13}F_{12}N$ requires: C, 36.1%; H, 3.3%; N, 3.5%; F, 57.1%).

Example 4

A mixture of N-methylmorpholine (9.28g, 92mmol) and hexafluoropropene (6.4g, 43mmol) was irradiated with gamma rays. The product was transferred under vacuum to give a volatile component (4.01g) and to leave a liquid (11.65g). Distillation in vacuo of the liquid gave N-(2,2,3,4,4,4-hexafluorobutyl)morpholine, (0.2g, 0.6%); b.p. 74°C at 11mmHg; (Found: C, 38.2%; H, 4.6%; N, 5.1%; F, 45.2%. $C_8H_{11}F_6NO$ requires: C, 38.2%; H, 4.4%; N, 5.6%; F, 45.4%); N-(2,2,3,4,4,4-hexafluorobutyl)-2-(1,1,2,3,3,3-hexafluoropropyl)-morpholine, (3,3g, 28%); b.p. 114°C at 11mmHg; (Found: C, 33.2%; H, 2.9%; N, 4.2%; F, 54.2%. $C_{11}H_{11}F_{12}NO$ requires: C, 32.9%; H, 2.7%; N, 3.5%; F, 56.8%).

Example 5

A mixture of N,N-dimethylformamide (20.33g, 278mmol) and hexafluoropropene (17.1g, 114mmol) was irradiated with gamma rays. The product was distilled in vacuo to give N-(2,2,3,4,4,4-hexafluorobutyl)-N-methylformamide, (50% by GLC, column K, 115°C; b.p. 38°C at 1mmHg); (Found: C, 32.3%; H, 3.5%; N, 6.5%; F, 51.1%. $C_6H_8F_6NO$ requires: C, 32.1%; H, 3.6%; N, 6.3%; F, 50.9%); N,N-dimethyl-2,2,3,4,4,4-hexafluorobutanamide (23% by GLC, column K, 115°C); b.p. 161°C (Siwoloboff); (Found: C, 32.6%; H, 3.3%; N, 6.6%; F, 50.4%. $C_6H_7F_6NO$ requires: C, 32.3%; H, 3.1%; N, 6.3%; F, 51.1%) and N-(2,2,3,4,4,4-hexafluorobutyl)-N-methyl-2,2,3,4,4,4-hexafluorobutanamide (7% by GLC, column K, 115°C); b.p. 196°C (Siwoloboff); (Found: C, 29.8%; H, 2.3%; N, 4.3%; F, 60.8%. $C_9H_7F_{12}NO$ requires: C 29.0%; H, 1.9%; N, 3.8%; F, 61.1%).

Example 6

A mixture of N,N-dimethylacetamide (10.34g, 119mmol) and hexafluoropropene (5.6g, 37mmol) was irradiated with gamma rays. The product was distilled in vacuo to give N-(2,2,3,4,4,4-hexafluorobutyl)-N-methylacetamide, (7.24g, 82%); b.p. 42°C at 1mmHg; (Found: C, 36.9%; H, 4.3%; N, 6.2%; F, 50.0%. $C_7H_9F_6NO$ requires: C, 35.4%; H, 3.8%; N, 5.9%; F, 48.1%).

Example 7

A mixture of N,N,N',N'-tetramethylurea (7.54g, 65mmol) and hexafluoropropene (6.6g, 44mmol) was irradiated with gamma rays. The product was distilled in vacuum to give N-(2,2,3,4,4,4-hexafluorobutyl)-N,N',N'-trimethylurea, (6.93g, 55%); b.p. 72°C at 1mmHg; m.p. 36-38°C; (Found: C, 36.1%; H, 4.3%; N, 10.4%; F, 41.3%. $C_8H_{12}F_6N_2O$ requires: C, 36.1%; H, 4.5%; N, 10.5%; F, 42.9%).

Example 8

A mixture of N,N,N',N'-tetramethylurea (2.85g, 24.6mmol) and hexafluoropropene (13.9g, 92.7mmol) was irradiated with gamma rays at 18°C. The product was transferred under vacuum to give alkene (5.71g) and a liquid (11.08g). The liquid was distilled in vacuo to give N-(2,2,3,4,4,4-hexafluorobutyl)-N,N',N'-trimethylurea, (10% by GLC, column K, 195°C); N,N'-di(2,2,3,4,4,4-hexafluorobutyl)-N,N'-dimethylurea, (53% by GLC); b.p. 101°C at 5mmHg; (Found: C, 32.0%; H, 2.7%; N, 6.3%; F, 54.0%. $C_{11}H_{12}F_{12}N_2O$ requires: C, 31.7%; H, 2.9%; N, 6.7%; F, 54.8%); N,N-di(2,2,3,4,4,4-hexafluorobutyl)-N',N'-dimethylurea, (9% by GLC), N,N,N'-tri(2,2,3,4,4,4-hexafluorobutyl)-N'-methylurea, (24% by GLC), spectra 57; and N,N,N',N'-tetra-(2,2,3,4,4,4-hexafluorobutyl)urea, (3% by GLC).

Example 9

A mixture of N,N,N',N'-tetramethylurea (0.9g, 7.8mmol) and hexafluoropropene (6.9g, 46mmol) and di-t-butyl peroxide (0.27g) was heated at 120°C for 65 hours to give N-(2,2,3,4,4,4-hexafluorobutyl)-N,N',N'-trimethylurea, (4% by GLC, column K, 195°C), N,N-di(2,2,3,4,4,4-hexafluorobutyl)-N',N'-dimethylurea (5%), N,N'-di(2,2,3,4,4,4-hexafluorobutyl)-N,N'-dimethylurea, (27%), N,N,N'-tri(2,2,3,4,4,4-hexafluorobutyl)-N'-methylurea, (40%) and N,N,N',N'-tetra-(2,2,3,4,4,4-hexafluorobutyl)urea (24%).

Example 10

A mixture of N-(2,2,3,4,4,4-hexafluorobutyl)-N,N',N'-trimethylurea (0.43g, 1.6mmol) and hexafluoropropene (4.3g, 29mmol) was irradiated with gamma rays. The product was transferred under vacuum to give alkene (3.79g) and a liquid (0.91g) composed of N-(2,2,3,4,4,4-hexafluorobutyl)-N,N',N'-trimethylurea, (2% by GLC, column K, 195°C), N, N,-di(2,2,3,4,4,4-hexafluorobutyl)-N',N'-dimethylurea, (2%), N,N'-di-(2,2,3,4,4,4-hexafluorobutyl)-N,N'-dimethylurea, (56%), N,N,N'-tri(2,2,3,4,4,4-hexafluorobutyl)-N'-methylurea, (32%), and N,N,N',N'-tetra(2,2,3,4,4,4-hexafluorobutyl)urea, (8%).

Example 11

A mixture of N,N-dimethylbenzamide (2.02g, 13.5mmol), hexafluoropropene (2.27g, 15mmol), and di-t-butyl peroxide (0.22g, 1.4mmol) was heated at 120°C for 18 hours. The product was transferred under vacuum to give alkene (1.6g) and a liquid (2.81g). The liquid was distilled in vacuo to give N-(2,2,3,4,4,4-hexafluorobutyl)-N-methylbenzamide, (30%); m.p. 60-62°C; (Found: C, 49.0%; H, 4.0%; N, 4.3%; F, 37.0%. $C_{12}H_{11}F_6NO$ requires: C, 48.2%; H, 3.7%; N, 4.7%; F, 38.1%).

Example 12

A mixture of N-methylpyrrolidin-2-one (10.1g, 102mmol) and hexafluropropene (5,1g, 34mmol) was irradiated with gamma rays. The product was distilled in vacuo to give N-methyl-5-(1,1,2,3,3,3-hexafluoropropyl)pyrrolidin-2-one, (5.1g, 60%); b.p. 83-85°C at 1mmHg; (Found: C, 39.1%; H, 3.9%; N, 5.5%; F, 43.8%. $C_8H_9F_6NO$ requires: C, 38.6%; H, 3.6%; N, 5.6%; F, 45.8%).

Example 13

A mixture of N-acetylpiperidine (5.9g, 46mmol) and hexafluoropropene (4.3g, 29mmol) was irradiated with gamma rays. The product was transferred under vacuum to give alkene (1.84g) and leave a liquid (8.31g). The liquid was distilled in vacuo to give N-acetyl-2-(1,1,2,3,3,3-hexafluoropropyl)piperidine, (40% by GLC, column K, 200°C); b.p. 43°C at 0.1mmHg; (Found: C, 44.1%; H, 5.0%; N, 5.0%; F, 39.8%. $C_{10}H_{13}F_6NO$ requires: C, 43.3%; H, 4.7%; N, 5.0%; F, 41.2%); and a mixture of di-adducts (17% by GLC/mass spectra, column K, 200°C).

Example 14

A mixture of N-acetylmorpholine (5,9g, 46mmol) and hexafluoropropene (3,5g, 23mmol) was irradiated with gamma rays. The product was transferred under vacuum to give alkene (2.26g) and leave a liquid (7.15g). The liquid was composed of adduct products (35% by GLC/mass spectra, column K, 195°C), but they were not separated.

Example 15

A mixture of N,N-dimethylacetamide (7.6g, 87mmol) and chlorotrifluoroethene (3.2g, 27mmol) was irradiated with gamma rays. The product (10.72g) was distilled in vacuo to remove starting materials then micro-distilled in vacuo to give N-methyl-N-3-chloro-2,2,3-trifluoropropyl)acetamide (76% by GLC, column K, 190°C); b.p. 225°C (Siwoloboff); (Found: C, 35.1%; H, 4.8%; N, 6.8%; F, 27,6%; Cl, 17.9%. $C_6H_9ClF_3NO$ requires: C, 35.4%; H, 4.4%; N, 6.9%; F, 28.0%; Cl, 17.4%); and leave a residue of a mixture of telomer adducts (22% by GLC/mass spectra, column K, 190°C).

Example 16

A mixture of N-methylpyrrolidin-2-one (8.9g, 90mmol) and chlorotrifluoroethene (6.1g, 52mmol( was irradiated with gamma rays. The product (14.46g) was distilled in vacuo to give N-methyl-5-(2-chloro-1,1,2-trifluoroethyl)pyrrolidin-2-one, (75% by GLC, column K, 190°C) b.p. 50-53°C at 5mmHg; (Found: C, 39.1%; H, 4.5%; N, 6.2%; F, 25.1%; Cl, 17.0%. $C_7H_9ClF_3NO$ requires: C, 39.0%; H, 4.2%; N, 6.5%; F, 26.4%; Cl, 16.5%); and a mixture of telomer adducts (24% by GLC/mass spectra, column K, 190°C).

Example 17

A mixture of N,N-dimethylacetamide (8.52g, 97mmol) and hexafluorocyclobutene (5.1g, 31mmol) was irradiated with gamma rays. The product was transferred under vacuum to give alkene (3.63g) and leave a liquid (9.73g). Water (50ml) was added to the liquid, the lower layer separated, and micro-distilled in vacuo to give N-(1,2,3,3,4,4-hexafluorocyclobutyl)-methyl-N-methylacetamide, (29%); b.p. 230°C (decomp) (Siwoloboff); (Found: C, 38.6%; H, 3.9%; N, 5.3%; F, 49.7%. $C_8H_9F_6NO$ requires: C, 38.6%; H, 3.6%; N, 5.6%; F, 45.8%).

Example 18

A mixture of N,N-dimethylacetamide (7.5g, 86mmol) and octafluorocyclopentene (6.8g, 32mmol) was irradiated with gamma rays. The product was transferred under vacuum to give alkene (4.86g) and leave a liquid (9.18g). Water (25ml) was added to the liquid, the lower layer separated, washed twice with water (2 x 25ml), and distilled in vacuo to give N-(1,2,3,3,4,4,5,5-octafluorocyclopentyl)methyl-N-methylacetamide, (28%); b.p. 54°C at 0.1mmHg; (Found: C, 36.7%; H, 2.6%; N, 1.2%; F, 49.9%. $C_9H_9F_8NO$ requires: C, 36.1%; H, 3.0%; N, 4.7%; F, 50.8%).

Example 19

A mixture of N,N-dimethylacetamide (0.96g, 11mmol) and decafluorocyclohexene (1.42g, 5mmol) was irradiated with gamma rays. The product was dissolved in the minimum amount of acetone, excess water added, and ether extracted (3 x 50ml). The ethereal solution was washed with water (3 x 20ml) and the solvent removed in vacuo. The remaining solid was re-crystallized (acetone) to give cis-N-(1,2,3,3,4,4,5,5,6,6-decafluorocyclohexyl)methyl-N-methylacetamide, (90%); m.p. 95-98°C; (Found: C, 34.4%; H, 2.9%; N, 4.0%; F, 57.6%. $C_{10}H_9F_{10}NO$ requires: C, 34.4%; H, 2.6%; N, 4.0%; F, 54.4%).

Example 20

A mixture of N-methylpyrrolidin-2-one (7.1g, 78mmol) and decafluorocyclohexene (6.5g, 25mmol) was irradiated with gamma rays. The product was washed three times with water to leave a lower layer which was heated in vacuo. The remaining viscous liquid (4.28g) was composed of at least three components (GLC, column K, 180°C) which could not be separated by distillation, sublimation, or thin layer chromatography.

Example 21

A mixture of N,N-methylacetamide (5.2g, 60mmol) and octafluorobut-2-ene (6.5g, 33mmol) was irradiated with gamma rays. The product was transferred under vacuum to give alkene (4.43g) and leave a liquid (7.02g). The liquid contained at least two adduct products (GLC/mass spectra, column K, 195°C) which could not be separated.

Example 22

A mixture of polyvinylpyrrolidinone (2.84g) and hexafluoropropene (12.3g 82mmol) was irradiated with gamma rays to a high dose (100Mrad). Excess alkene (9.51g) was transferred under vacuum to leave a solid (5.08g). The solid was refluxed with water for 1 hour, washed with water, and dried in vacuo. (Found: C, 33.2%; H, 1.2%; N, 3.4%; F, 52.1%).

**Claims**

1. An organic fluorine compound of the formula:

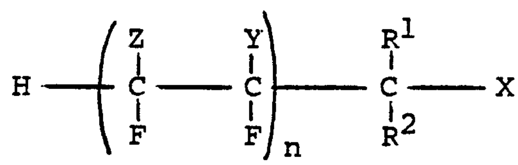

wherein X represents an isocyanato, optionally substituted amino or optionally substituted amido group;
$R^1$ represents hydrogen or an optionally substituted alkyl radical;
$R^2$ represents hydrogen or an alkyl radical;

Y represents F and Z represents F, Cl or $CF_3$ or Y and Z together form a $-(CF_2)_m-$ chain wherein m represents an integer from 2 to 4, and
n represents a positive integer which must be 1 when Z is Cl or $CF_3$.

2. An organic fluorine compound according to claim 1 wherein X is an isocyanato group and $R^1$ is an isocyanatoalkyl group.

3. An organic fluorine compound according to claim 1 having the formula:

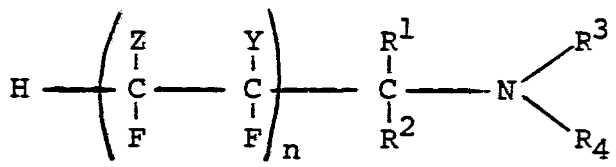

wherein $R^1$, $R^2$, Y, Z and n have the meanings given in claim 1, $R^3$ and $R^4$ represent optionally substituted alkyl radicals or $R^3$ and $R^4$ together, or $R^3$ and $R^1$ together, represent an alkylene chain which may be interrupted by a hetero-atom.

4. An organic fluorine compound according to claim 1 having the formula:

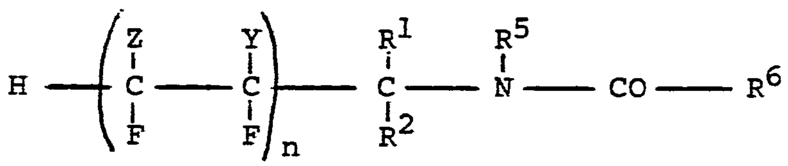

wherein $R^1$, $R^2$, Y, Z and n have the meanings given in claim 1, $R^5$ represents a hydrogen or an optionally substituted alkyl radical and $R^6$ represents an optionally substituted alkyl, aryl or dialkylamino radical or $R^5$ and $R^6$ together, or $R^5$ and $R^1$ together, represent an alkylene chain which may be interrupted by a hetero-atom.

5. A method for the preparation of an organic fluorine compound as defined in claim 1 which comprises the free radical addition of a compound of the formula:

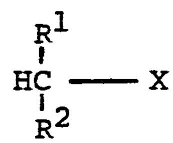

and a fluoro-olefin of the formula:

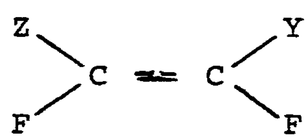

wherein X, $R^1$, $R^2$, Y and Z have the meanings given in claim 1.

6. A method according to claim 5 wherein the fluoro-olefin is hexafluoropropene.

European Patent Office

# EUROPEAN SEARCH REPORT

Application number

EP 87 30 2388

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 019 261 (I. PASCAL)<br>* Whole document *<br>--- | 1,3-6 | C 07 C 119/042<br>C 07 C 103/30<br>C 07 C 127/15<br>C 07 D 207/26<br>C 07 D 211/18<br>C 07 D 265/30 |
| X | US-A-2 646 449 (J.E. CARNAHAN)<br>* Whole document *<br>--- | 1 | |
| A | US-A-3 816 286 (R.N. HASZELDINE)<br>* Column 2, line 20 - column 3, line 68; example 16 *<br>--- | 1,4-6 | |
| A | US-A-2 706 733 (T.S. REID)<br>* Column 1, lines 15-26; column 2, lines 18-40 *<br>--- | 1,2 | |
| A | GB-A-1 231 952 (FMC)<br>* Claims *<br>--- | 1,4 | TECHNICAL FIELDS SEARCHED (Int. Cl.4) |
| A | DE-C- 924 515 (DU PONT)<br>* Claims; page 8, column 1, lines 4-33 *<br>----- | 1,3-5 | C 07 C 103/00<br>C 07 C 119/00<br>C 07 C 127/00<br>C 07 D 211/00 |

The present search report has been drawn up for all claims

| Place of search | Date of completion of the search | Examiner |
|---|---|---|
| THE HAGUE | 07-07-1987 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82